# EUROPEAN PATENT SPECIFICATION

(11) **EP 3 241 556 B1**
(45) Date of publication and mention of the grant of the patent: **10.03.2021**
(21) Application number: 17177585.1
(22) Date of filing: 13.11.2009
(51) Int. Cl.: A61K 35/19, A61P 9/00, A61P 9/10, A61P 17/02, A61L 15/40, A61L 26/00, A61N 1/32

(54) **ACTIVATION AND AGGREGATION OF HUMAN PLATELETS AND FORMATION OF PLATELET GELS BY NANOSECOND PULSED ELECTRIC FIELDS**
AKTIVIERUNG UND AGGREGATION VON MENSCHLICHEN THROMBOZYTEN UND BILDUNG VON THROMBOZYTENGEL DURCH NANOSEKUNDEN-GEPULSTE ELEKTRISCHE FELDER
ACTIVATION ET AGRÉGATION DE PLAQUETTES HUMAINES ET FORMATION DE GELS DE PLAQUETTES PAR DES CHAMPS ÉLECTRIQUES PULSÉS DE L'ORDRE DE LA NANOSECONDE (NSPEF)

(30) Priority: 13.11.2008 US 114363 P
(43) Date of publication of application: 08.11.2017
(62) Divisional of application: 09826853.5
(73) Proprietor: Eastern Virginia Medical School, Norfolk, VA 23507 (US); Old Dominion University Research Foundation, Norfolk, VA 23508-0369 (US)
(72) Inventor: HARGAVE, Barbara, Y., Norfolk, VA 23510 (US); SCHOENBACH, Karl, H., Norfolk, VA 23510 (US); BLACKMORE, Peter, F., Virginia Beach, VA 23455 (US); BEEBE, Stephen, J., Norfolk, VA 23508 (US)
(74) Representative: Atkins, James Gordon John

(56) References cited:
- EP-A1- 1 674 115
- US-A1- 2006 161 221
- US-A1- 2007 014 784
- XIAO S ET AL: "Pulsed Power for Wound Healing", IEEE INTERNATIONAL POWER MODULATORS AND HIGH VOLTAGE CONFERENCE, PROCEEDINGS OF THE 2008, IEEE, PISCATAWAY, NJ, USA, 27 May 2008 (2008-05-27), pages 69-72, XP031403856, ISBN: 978-1-4244-1534-2
- ZHANG ET AL: "Nanosecond pulse electric field (nanopulse): A novel non-ligand agonist for platelet activation", ARCHIVES OF BIOCHEMISTRY AND BIOPHYSICS, ACADEMIC PRESS, US, vol. 471, no. 2, 23 December 2007 (2007-12-23), pages 240-248, XP022503652, ISSN: 0003-9861
- BEEBE S J ET AL: "Nanosecond pulsed electric fields mimic natural cell signal transduction mechanisms", POWER MODULATOR SYMPOSIUM, 2004 AND 2004 HIGH-VOLTAGE WORKSHOP. CONFER ENCE RECORD OF THE TWENTY-SIXTH INTERNATIONAL SAN FRANCISCO, CALIFORNIA, USA MAY 23-26, 2004, PISCATAWAY, NJ, USA,IEEE, 23 May 2004 (2004-05-23), pages 220-223, XP010801177, ISBN: 978-0-7803-8586-3
- BEEBE S J ET AL: "Dynamic effects and applications for nanosecond pulsed electric fields in cells and tissues", PROGRESS IN BIOMEDICAL OPTICS AND IMAGING - PROCEEDINGS OF SPIE - ADVANCED BIOMEDICAL AND CLINICAL DIAGNOSTIC SYSTEMS III 2005 SPIE US, vol. 5692, 2005, pages 260-269, XP002686212, DOI: DOI:10.1117/12.604449
- SCHOENBACH K H ET AL: "Bioelectric Effects of Intense Nanosecond Pulses", IEEE TRANSACTIONS ON DIELECTRICS AND ELECTRICAL INSULATION, IEEE SERVICE CENTER, PISCATAWAY, NJ, US, vol. 14, no. 5, 1 October 2007 (2007-10-01), pages 1088-1109, XP011193462, ISSN: 1070-9878, DOI: 10.1109/TDEI.2007.4339468
- STASHAK T S ET AL: "Update on wound dressings: Indications and best use", CLINICAL TECHNIQUES IN EQUINE PRACTICE, W.B. SAUNDERS, vol. 3, no. 2, 1 June 2004 (2004-06-01), pages 148-163, XP004768815, ISSN: 1534-7516, DOI: 10.1053/J.CTEP.2004.08.006

## Description

### BACKGROUND OF THE INVENTION

Electric fields can be used to manipulate cell function in a variety of ways. One specific cell mechanism that can be affected by electric fields is calcium mobilization within a cell. Calcium signaling, an important cell function, is responsible for a variety of cellular responses and actions. The release of internally stored calcium can stimulate responses to agonists, activate growth and respiration, cause the secretion of neurotransmitters, activate transcription mechanisms, cause the release of a variety of hormones, produce muscle contractions, and initiate release of key factors in the apoptosis pathway (Berridge, M.J., Bootman, M.D., Lipp, P. (1998) Nature. 395, 645-648). This calcium mobilization also triggers the influx of calcium from the external medium into the cell as a means of further propagating calcium signals and also replenishing depleted pools of calcium. Electric fields can be used to manipulate the movement of ions, such as calcium, in order to study calcium signaling.

One application of this calcium increase is to activate platelets and cause them to aggregate *in vitro* and *in vivo.* Platelet activation / aggregation is important for preventing blood loss during traumatic injury or surgery by forming a hemostatic plug at the site of injury. At present, treatment with thrombin, known to increase intracellular calcium in human platelets, is used to control slow bleeding at sites of injury. Thrombin treatment includes the topical application of bovine or recombinant thrombin, or the use of platelet gels in which autologous platelets are treated with bovine thrombin and added to the surgical site (Brissett and Hom (2003) Curr. Opin. Otolaryngol. Head Neck Surgery 11, 245-250; Man et al., (2001) Plast. Reconstr. Surg. 107, 229-237; Saltz (2001) Plast. Reconstr. Surg. 107, 238-239; Bhanot and Alex (2002) Facial Plast. Surg. 18, 27-33). However, the use of animal products could cause allergic reactions or cause possible contamination of platelet rich plasma (PRP) with infectious agents. The use of recombinant thrombin or a peptide that mimics thrombin action could be used as an alternative to animal-derived thrombin; however, this type of treatment is expensive and could also give rise to allergic reactions.

Since calcium signaling plays such an important role in so many cellular functions, there remains a need to further examine this signaling mechanism and explore ways to manipulate calcium signaling pathways for therapeutic purposes. For example, there is a need to develop methods of activating calcium-mediated cell functions, including aggregation of human platelets, for therapeutic purposes, such as wound healing. These and various other needs are addressed, at least in part, by one or more embodiments of the present invention.

### SUMMARY OF THE INVENTION

In a first aspect, the invention provides a platelet gel for use in a method of treating systolic dysfunction and/or diasystolic dysfunction of a heart in a subject, wherein the platelet gel is obtained by a method comprising:
concentrating platelets; and
activating the concentrated platelets by applying at least one electrical pulse to the concentrated platelets,
wherein the electrical pulse has a duration of at least about 100 picoseconds and less than about 1 microsecond and an electric field strength of at least about 10 kV/cm and less than about 350 kV/cm.
In a second aspect, the invention provides, a bandage or a tissue repair matrix for use in a method of treating systolic dysfunction and/or diasystolic dysfunction of a heart in a subject, the bandage or tissue repair matrix comprising a platelet gel, the platelet gel being obtained by a method comprising:
concentrating platelets; and
activating the concentrated platelets by applying at least one electrical pulse to the concentrated platelets,
wherein the electrical pulse has a duration of at least about 100 picoseconds and less than about 1 microsecond and an electric field strength of at least about 10 kV/cm and less than about 350 kV/cm.

Embodiments of the invention are set out in the dependent claims.

One or more aspects of the disclosure provide a method for inducing calcium mobilization in a cell. The method comprises applying at least one electrical pulse to one or more cells, whereby calcium is mobilized in the cells. According to at least one embodiment, the electrical pulse comprises at least one nanosecond pulsed electric field (nsPEF). The at least one nsPEF has a pulse duration of at least about 100 picoseconds and no more than about 1 microsecond and an electric field strength of at least about 10 kV/cm and no more than about 350 kV/cm. In one or more embodiments of the invention, calcium influx into the cells occurs.

In one or more aspects of the disclosure, the cells are human platelets, whereby activation and aggregation of the platelets is induced.

The disclosure also provides a method for increasing intracellular calcium in cells comprising applying at least one nsPEF to the cells, whereby intracellular calcium in the cells is increased. The at least one nsPEF has a pulse duration of at least about 100 picoseconds and no more than about 1 microsecond and an electric field strength of at least about 10 kV/cm and no more than about 350 kV/cm. In one or more embodiments, the cells are human platelets, whereby activation and aggregation of the platelets is induced.

Also provided in the disclosure is a method for activating and aggregating human platelets comprising applying at least one nsPEF to the platelets, whereby the platelets are activated and induced to form aggregates. The at least one nsPEF has a pulse duration of at least about 100 picoseconds and no more than about 1 microsecond and an electric field strength of at least about 10 kV/cm and no more than about 350 kV/cm. In one aspect, the at least one nsPEF has a pulse duration of about 10 nanoseconds and an electric field strength of about 125 kV/cm. In another aspect, the at least one nsPEF has a pulse duration of about 60 nanoseconds and an electric field strength of about 30 kV/cm. In another embodiment, the at least one nsPEF has a pulse duration of 300 nanoseconds and an electric field strength of 30 kV/cm. The platelets may be suspended in a medium or included in a tissue or in a natural or synthetic tissue repair matrix, such as but not limited to bioresorbable collagen scaffold or matrix, or incorporated into bandage or wound closure devices. In other embodiments, activated platelets are applied or incorporated into bandages or sutures that may be applied to a wound.

The disclosure also provides a method of treating an injury, trauma, or the loss of blood in a subject, comprising applying at least one nsPEF to autologous platelets, whereby the platelets are activated and induced to form aggregates. The activated and aggregated platelets are then applied to the site of injury, trauma, or blood loss. The at least one nsPEF has a pulse duration of at least about 100 picoseconds and no more than about 1 microsecond and an electric field strength of at least about 10 kV/cm and no more than about 350 kV/cm. The blood loss in a subject may be related to a bleeding disorder resulting from inactive platelets or low platelet counts. The blood loss may also be related to a platelet disorder such as congenital afibrinogenemia, Glanzmann's thrombasthenia, gray platelet syndrome, and Hermansky-Pudlak syndrome.

As a further embodiment for the preparation of activated platelet aggregations, at least another aspect of the disclosure provides a method for preparing platelet gels comprising human platelets comprising applying at least one nsPEF to the platelets, whereby the platelets are activated. The at least one nsPEF has a pulse duration of at least about 100 picoseconds and no more than about 1 microsecond and an electric field strength of at least about 10 kV/cm and no more than about 350 kV/cm. In one aspect, the at least one nsPEF has a pulse duration of about 10 nanoseconds and an electric field strength of about 125 kV/cm. In another aspect, the at least one nsPEF has a pulse duration of about 60 nanoseconds and an electric field strength of about 30 kV/cm. In another embodiment, the at least one nsPEF has a pulse duration of 300 nanoseconds and an electric field strength of 30 kV/cm. The platelets may be suspended in a medium or included in a tissue or in a natural or synthetic tissue repair matrix, such as but not limited to bioresorbable collagen scaffold or matrix or incorporated into a bandage or wound closure devices.

At least another aspect of the disclosure provides a method for treating an injury, trauma, or the loss of blood in a subject, comprising applying platelets at or near the site of injury, trauma, or blood loss, whereby the platelets are activated and induced to form gels through application of at least one nsPEF. The at least one nsPEF has a pulse duration of at least about 100 picoseconds and no more than about 1 microsecond and an electric field strength of at least about 10 kV/cm and no more than about 350 kV/cm.

At least another aspect of the disclosure provides a method for treating and/or preventing infection at the site of an injury, trauma, or the loss of blood in a subject, comprising applying platelets at the site of injury, trauma, or blood loss, whereby the platelets are activated and induced to form gels through application of at least one nsPEF. The at least one nsPEF has a pulse duration of at least about 100 picoseconds and no more than about 1 microsecond and an electric field strength of at least about 10 kV/cm and no more than about 350 kV/cm. In another embodiment, the at least one nsPEF has a pulse duration of 300 nanoseconds and an electric field strength of 30 kV/cm.

At least another aspect of the disclosure provides a method for altering the acute changes in systolic and diastolic pressures in the left ventricle of the heart after an ischemic event, such as ischemia-reperfusion, whereby the platelets are activated and induced to form gels through application of at least one nsPEF and injected into the myocardial tissue. The at least one nsPEF has a pulse duration of at least about 100 picoseconds and no more than about 1 microsecond and an electric field strength of at least about 10 kV/cm and no more than about 350 kV/cm. In another embodiment, the at least one nsPEF has a pulse duration of 300 nanoseconds and an electric field strength of 30 kV/cm.

At least another aspect of the disclosure envisions the application of activated platelets to the surface of the heart, whereby the platelets are activated and induced to form gels through application of at least one nsPEF. The at least one nsPEF has a pulse duration of at least about 100 picoseconds and no more than about 1 microsecond and an electric field strength of at least about 10 kV/cm and no more than about 350 kV/cm. In another embodiment, the at least one nsPEF has a pulse duration of 300 nanoseconds and an electric field strength of 30 kV/cm.

At least another aspect of the present disclosure provides a bandage or wound closure device, such as a suture, containing an application or suspension of activated platelet gel, whereby the platelets are activated and induced to form gels through application of at least one nsPEF. Various embodiments envision activation of platelets before and after application of the platelet get to the bandage, where the at least one nsPEF has a pulse duration of at least about 100 picoseconds and no more than about 1 microsecond and an electric field strength of at least about 10 kV/cm and no more than about 350 kV/cm

### BRIEF DESCRIPTION OF THE FIGURES

Figure 1 shows the effects of nsPEF pulses (10 ns and 125 kV/cm) on intracellular calcium in human platelets. Increases in intracellular calcium were shown to be dependent on the number of nsPEF pulses applied, with ten pulses causing a two-fold increase in calcium.
Figure 2 shows that calcium is mobilized from intracellular stores in the absence of extracellular calcium, followed by capacitive calcium influx when calcium is added to the extracellular media. Fura-2 loaded cells were pulsed in the absence of extracellular calcium and the calcium concentration was determined in a fluorometer. After 2-3 minutes, calcium was added to the extracellular media as the readings continued.
Figure 3 shows that there is a pulse-dependent increase in platelet aggregation when platelets are pulsed at 125 kV/cm for 10 ns. Platelets were placed in the aggregometer, a baseline light transmittance measured, calcium was added at 15 seconds, then platelets were removed at 30 seconds into the pulsing cuvette. The platelets were pulsed 1, 2, 5 or 10 times for 10 ns each at 125 kV/cm. The platelets were then placed back into the aggregometer and aggregation measured. The 10 pulse treatment produced an aggregation response similar to that observed with 0.02 units/ml thrombin.
Figure 4 shows normalized systolic pressure in the left ventricle of an isolated rabbit heart following 30 minutes of global ischemia and during the period of reperfusion. The data are stated as the mean ± SD. * α =0.1, p<0.01, saline versus thrombin and nsPEF.
Figure 5 shows normalized diastolic pressure in the left ventricle of an isolated rabbit heart following 30 minutes of global ischemia and during the period of reperfusion. The data are stated as the mean ± SD. * (α =0.1, p<0.01, saline versus thrombin and nsPEF).
Figure 6 shows normalized work function in the left ventricle of an isolated rabbit heart following 30 minutes of global ischemia and during the period of reperfusion. The data are stated as the mean ± SD.
Figure 7 shows normalized pulse pressure in the left ventricle of an isolated rabbit heart following 30 minutes of global ischemia and during the period of reperfusion. The data are stated as the mean ± SD. * (α =0.1, p<0.05 thrombin and nsPEF versus saline).
Figure 8 shows representative examples of growth of Stappylococcus Aeurus in the presence or absence of platelet gel prepared with nsPEF or bovine thrombin.
Figure 9 shows the growth results of Stappylococcus Aeurus in the presence or absence of platelet gel prepared with nsPEF or bovine thrombin.
Figures 10A-D show the *in vivo* response in a nsPEF activated platelet gel or saline treated heart.

Figure 10E shows duration of left ventricular relaxation (DREL) 14 days post AMI in response to dobutamine stress.
Figure 10F shows duration of left ventricular relaxation (DREL) 14 days post AMI in responses to dobutamine stress.
Figures 11A and 11B show heart tissue without nsPEF activated platelet gel treatment and with nsPEF activated platelet gel treatment, respectively.

### DETAILED DESCRIPTION OF THE INVENTION

For the purposes of promoting an understanding of the principles of the invention, reference will now be made to preferred embodiments and specific language will be used to describe the same. It will nevertheless be understood that no limitation of the scope of the invention is thereby intended. Rather, such alterations and further modifications of the invention, and such further applications of the principles of the invention as illustrated herein, as would be contemplated by one having skill in the art to which the invention relates are intended to be part of the present invention.

For example, features illustrated or described as part of one embodiment can be used on other embodiments to yield a still further embodiment. Additionally, certain features may be interchanged with similar devices or features not mentioned yet which perform the same or similar functions. It is therefore intended that such modifications and variations are included within the totality of the present inventionprovided they are within the scope of the claims.

One or more embodiments of the present disclosure are directed to a method of inducing calcium mobilization in a cell using nanosecond pulsed electric fields ("nsPEFs"). "Calcium mobilization" as used herein is defined as the release of internally stored calcium in cells and/or the influx of calcium from the external medium into the cell. In one or more embodiments of the invention, calcium mobilization leads to an increase in intracellular free calcium levels of cells.

An "nsPEF" or "nanosecond pulsed electric field" as used herein is defined as an electric pulse in the nanosecond range (about 100 picoseconds to about 1 microsecond) with electric field intensities from about 10 kV/cm to about 350 kV/cm. For delivery of nsPEFs to cells, any apparatus equipped with a pulse generator that can deliver short electrical pulses of pulse duration of at least about 100 picoseconds and no more than about 1 microsecond, and of electric field strength of at least about 10kV/cm and no more than about 350 kV/cm, may be used. In another aspect of the invention, the pulse generator can deliver short electrical pulses of pulse duration of at least about 100 picoseconds and no more than about 1 microsecond, and of electric field strength of at least about 10kV/cm and no more than about 30 kV/cm. In another aspect of the invention, the pulse generator can deliver short electrical pulses of pulse duration of at least about 100 picoseconds and no more than about 1 microsecond, and of electric field strength of at least about 10kV/cm and no more than about 125 kV/cm. In another aspect of the invention, the pulse generator can deliver short electrical pulses of pulse duration of at least about 10 nanoseconds and no more than about 100 nanoseconds, and of electric field strength of at least about 10kV/cm and no more than about 30 kV/cm. In another aspect of the invention, the pulse generator can deliver short electrical pulses of pulse duration of at least about 10 nanoseconds and no more than about 100 nanoseconds, and of electric field strength of at least about 10kV/cm and no more than about 125 kV/cm. In another aspect of the invention, the pulse generator can deliver short electrical pulses of pulse duration of about 10 nanoseconds and an electric field strength of about 125kV/cm. In another aspect of the invention, the pulse generator can deliver short electrical pulses of pulse duration of about 60 nanoseconds and an electric field strength of about 30kV/cm.

Notably, the nsPEFs are distinct from electroporation pulses based on their temporal and electrical characteristics, as well as their effects on intact cells and tissues. For comparative purposes, electroporation pulses and nsPEFs, respectively, exhibit different electric field strength (*1-5 kV*/*cm vs. 10-350 kV*/*cm*)*;* different pulse durations (*0.1-20 milliseconds vs. 1-300 nanoseconds*)*;* different energy densities (*joules*/*cc vs. millijoules*/*cc*) and different power (*500W vs. 180MW*). Thus, nsPEFs can be five to six orders of magnitude shorter with electric fields and power several orders of magnitude higher and energy densities considerably lower than electroporation pulses. In addition to the unique short duration and rapid rise time, nsPEFs are exceptional because they are very low energy and extremely high power. Stemming from these differences, as the pulse duration decreases, nsPEFs bypass the plasma membrane and target intracellular structures such as the mitochondria, endoplasmic reticulum, Golgi apparatus, nucleus, or any intracellular store, leaving the plasma membrane intact. These pulses have effects that are unexpectedly different than those of electroporation pulses because, when the pulse duration is short enough and the electric field intensity is high enough, intracellular structures are targeted. The effects of nsPEFs on cells differ depending on the cell type, pulse duration and rise-time, electric field intensity, and/or other factors.

In addition, nsPEFs and electroporation pulses have different effects on cells. For example, Jurkat cells exposed to classical electroporation pulses (e.g. 100µs) exhibited immediate propidium iodide ("PI") uptake, but when exposed to 60 or 300ns they took up PI at much later times, consistent with apoptosis induction (Deng, J., et al. (2003), Biophys. J. 84, 2709-2714). Furthermore, in contrast to classical electroporation effects where larger cells are more readily electroporated than smaller cells, nsPEFs have greater plasma membrane effects on smaller cells (e.g. T-cells) than larger ones (e.g. monocytes). Under conditions that are independent of plasma membrane electroporation, nsPEFs have been shown to alter signal transduction mechanisms that determine cell fate. Using nsPEFs, it is possible to trigger apoptosis (Beebe, S.J., et al. (2002), IEEE Trans. Plasma Sci. 30:1 Part 2, 286-292; Beebe, S.J., et al. (2003), FASEB J (online, June 17, 2003) 10.1096//fj.02-0859fje; Vernier, P.T., et al. (2003), Biochem. Biophys. Res. Comm. 310, 286-295). nsPEFs induced several well-characterized apoptosis markers including intact plasma membranes, annexin-V-FITC binding, caspase activation, cell shrinkage, cytochrome c release into the cytoplasm, and ultimately, a late secondary necrosis as defined by rupture of the plasma membrane *in vitro* in the absence of phagocytosis (Beebe et al., 2003).

The apparatus for delivery of nsPEFs is also equipped with a high voltage power supply and with a means for directing the nsPEFs to the target cells *in vitro* or *in vivo.* Suitable means for directing the nsPEFs will preferably allow high voltage, short duration electrical pulses in the nanosecond range, for example, in cell suspensions or within tissues. Examples include an electrode system, such as needles or needle arrays. In one or more embodiments of the invention, the nsPEFs are applied to cells suspended in a medium. In other embodiments, the nsPEFs are applied to autologous platelets, thereby activating the platelets and inducing them to form aggregates, and the activated and aggregated platelets are then applied to a site of injury, trauma, or blood loss. In other embodiments, the nsPEFs are applied directly to the site where bleeding is occurring.

The nsPEF pulses of the present invention can be administered to the cells by means of a pulse generator, such as the generator previously described in U.S. Patent No. 6,326,177 and Beebe et al. FASEB J. 17, 1493-1495 (2003). Prior to the above-described pulse generator, the application of these high frequency intracellular effects had been limited due to the difficulty of generating large intracellular electric fields on a time scale that is comparable to or even less than the charging time of the surface. However, as described in U.S. Patent No. 6,326,177 and Beebe et al. (2003), the present inventors developed technology for generating high voltage, short duration electrical pulses that make it possible to produce electric pulses in the nanosecond range with voltage amplitudes adequate to generate electric fields near MV/cm in suspensions of cells or within tissues (Mankowski, J., Kristiansen, M. (2000) IEEE Trans Plasma Science 28:102-108). Because of their nanosecond duration, the average energy transferred to the cells/tissues by these pulses is theoretically negligible, resulting in electrical effects without accompanying thermal effects.

The electric field strength (or electric field intensity) of the nsPEF pulse to be applied to cells is the applied voltage divided by the distance between the electrodes, and is generally at least about 10kV/cm, but should not exceed the breakdown field of the suspension or tissue which includes the cells. The breakdown field increases with decreasing pulse duration, and can be experimentally determined. Under the conditions commonly employed in the present invention, however, the breakdown field generally does not exceed 500kV/cm. In one or more aspects of the invention, electric field pulses which have durations of about 100 picoseconds to about 1 microsecond have electric field strengths of about 10kV/cm to about 350kV/cm.

To minimize the potential effects on the bulk temperature of the medium ("thermal effects"), the electric field pulses generally have a rapid rise time and short duration. The pulses should preferably be less than one microsecond, but more than about 100 picoseconds in duration. In one or more aspects of the invention, a pulse duration is about 1 nanosecond to about 300 nanoseconds. The optimum pulse duration will vary depending on the cell type, tissue type, and desired treatment, among other factors.

The number of nsPEF pulses to be applied to the cells may be that sufficient to induce calcium mobilization. This number may vary based on a variety of factors included the intended effect, the mode of administration of the nsPEFs, and the cells to be treated. In one aspect of the invention, one nsPEF is applied to the cells to induce calcium mobilization. In another aspect of the invention, at least one nsPEF is applied to the cells. In another aspect of the invention, at least two nsPEFs are applied to the cells. In another aspect of the invention, at least five nsPEFs are applied to the cells. In another aspect of the invention, at least ten nsPEFs are applied to the cells. In yet another aspect of the invention, 1-10 nsPEFs are applied to the cells.

One or more embodiments of the disclosure are directed to methods of activating and aggregating platelets through the use of nsPEFs. In human platelets, nsPEF-induced calcium mobilization was found to induce platelet activation and aggregation, thereby providing a mechanism to clot blood and heal wounds. Accordingly, in one embodiment, the disclosure is directed to a method of activating and aggregating platelets comprising the application of nsPEF pulses to the cells to induce platelet activation and/or platelet aggregation. In another embodiment, the subject matter disclosed herein may be used in any clinical situation where there is any site of injury, trauma, or blood loss, either induced during surgery or as the result of trauma that results in the loss of blood. In some embodiments, the invention involves electrically pulsing autologous platelets outside the body of the animal to induce platelet activation and platelet aggregation, and applying the activated platelet aggregates or gels at the site of injury, trauma, or blood loss.

In further embodiments, autologous platelets are treated with nsPEFs to form activated platelet gels before application at the site of injury, trauma or blood loss. Platelet gel may be prepared by known methods, such as, for example, those described by Harvest Technologies. For example, platelet gel was prepared by having sixty ml of blood withdrawn from a donor using a sterile syringe containing 3 ml of ACD-A anticoagulant (Terumo Cardiovascular System, Ann Arbor, MI). A SmartPRep@-2 Platelet Concentrate System and a sterile processing disposable pack was used to prepare platelet gel. The processing disposable was placed into a centrifuge and centrifuged for 14 minutes to separate the blood components from the plasma (Harvest Technology). According to one embodiment, Harvest Technology System concentrator was used to concentrate platelets in whole blood 4-7 times providing a platelet concentrate, for example, between 1180 X 10³/µl and 2065 X 10³/µl. Platelet Poor Plasma ("PPP") was used to resuspend the concentrated platelets to a final volume of 7 ml. An electrical pulse (300 ns) was applied to suspended platelets in electroporation cuvettes (electrode gap of 0.2 cm, plate electrodes of aluminum, area of 1cm²). According to one embodiment, platelet gels may be activated using nsPEFs of 300 ns duration and an electric field of 30 kV/cm in the presence of 10 mM calcium.

In further embodiments, activated platelet gels according to the disclosure are placed at the site of an injury, trauma, or the loss of blood in a subject to treat and/or prevent infection at the site. In such embodiments, the activated platelet gel inhibits the growth of Staphylococcus Aureus at the site, though the prevention of growth of other bacterial strains is envisioned in further embodiments.

In further embodiments, activated platelet gels produced by treatment with nsPEF may be used to treat injured heart tissue. For example, according to one embodiment, activated platelet gels produced by treatment with nsPEF may be injected directly into the myocardium for treatment of acute changes in systolic and diastolic pressures in the heart after ischemia-reperfusion, as in the case of a heart suffering or that has suffered myocardial infarction. In this embodiment, activated platelet gels produced by nsPEF improve ventricular filling and maintain or improve cardiac output in contrast to similar heart tissue treated with saline.

Reference will now be made to specific examples illustrating the use of nsPEFs in inducing calcium mobilization and activating platelet gels. It is to be understood that the examples are provided to illustrate applications of the preferred embodiments and that no limitation of the scope of the invention is intended thereby.

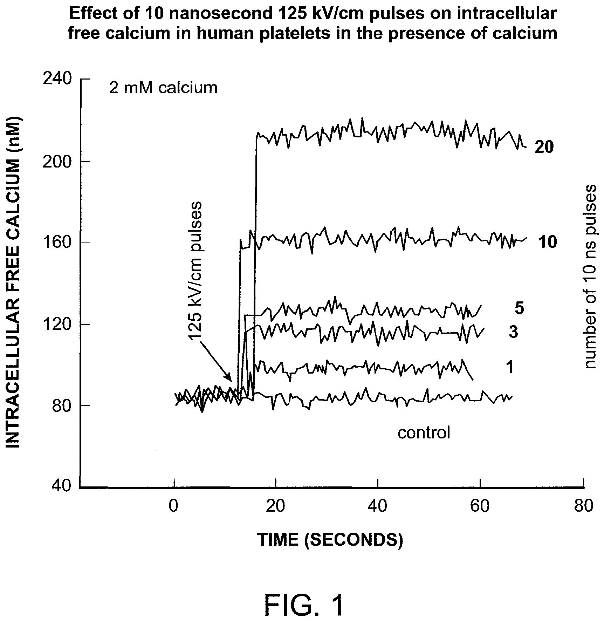

Example 1: The effect of nsPEFs in increasing intracellular calcium in human platelets: nsPEFs increase intracellular calcium in human platelets in a pulse-dependent manner, as shown in Figure 1. nsPEF pulses (10ns and 125 kV/cm) were applied to human platelets in experiments conducted in the presence of extracellular calcium. Calcium concentration was determined using Fura 2 as a quantifiable calcium indicator in a fluorometer. Increases in intracellular calcium were shown to be dependent on the pulse number (Figure 1). Ten pulses caused a two-fold increase in calcium. The calcium response was also found to depend on the electric field condition. Specifically, longer pulses and lower electric fields (e.g. 60ns and 30 kV/cm) produced more robust increases in calcium. Under these conditions, ten pulses caused a 3-fold increase in calcium. The kinetics of the calcium mobilization in response to nsPEF is different than the response to thrombin.

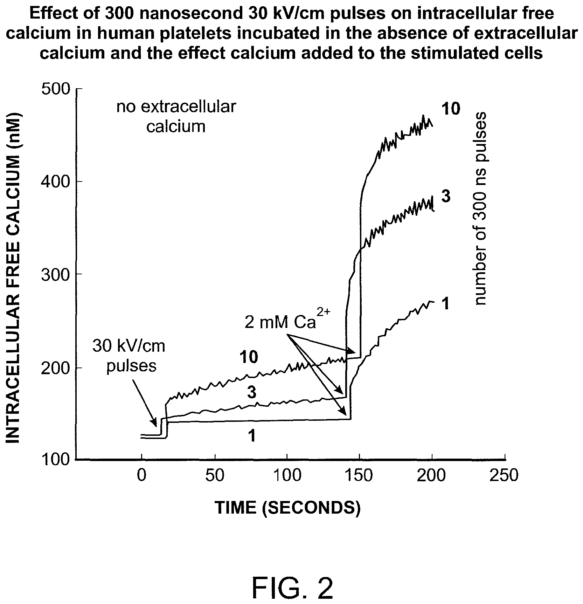

Calcium is mobilized from intracellular stores in the absence of extracellular calcium followed by capacitative calcium influx when calcium is added to the extracellular media, as shown in Figure 2. Cells were loaded with the calcium indicator Fura-2, pulsed in the absence of extracellular calcium, and the calcium concentration was determined in a fluorometer. After 2-3 minutes, calcium was added to the extracellular media as the readings continued. The initial calcium mobilization was determined to come from intracellular stores of calcium. Studies with human HL-60 cells indicate that this calcium is released into the cytoplasm from the endoplasmic reticulum (ER) (White et al., 2004). When calcium was added to the extracellular media, a capacitative calcium influx through store-operated calcium channels in the plasma membrane (PM) was observed. This mimics the response to thrombin, which is known to release calcium from the ER, followed by capacitative calcium entry through store-operated channels in the PM. Similar results were observed with nsPEF-treated HL-60 cells in comparison with purinergic agonists (White et al., 2004) and in nsPEF-treated Jurkat cells in comparison with CD-3 stimulation. This is in contrast to results from studies with nsPEF-treated polymorphonuclear leukocytes (PMNs), where calcium entry is not through store-operated calcium channels in the PM. (Buescher et al., poster Bioelectromagnetics Society meeting June 2004).

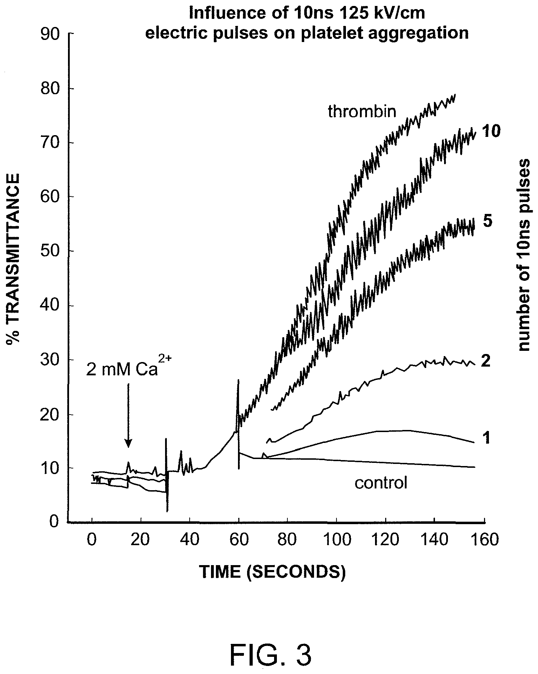

nsPEF can cause platelets to aggregate in a manner similar to that observed with thrombin (Figure 3). In particular, a pulse dependent increase in platelet aggregation was observed when platelets are pulsed at 125 kV/cm for 10 ns. Platelets were placed in the aggregometer, a baseline light transmittance measured, calcium was added at 15 seconds, then platelets were removed at 30 seconds into the pulsing cuvette. The platelets were pulsed 1, 2, 5 or 10 times for 10 ns each at 125 kV/cm. The platelets were then placed back into the aggregometer and aggregation measured. The 10 pulse treatment produced an aggregation response similar to that observed with 0.02 units/ml thrombin. When the duration of the nsPEF is increased, lower electric fields are needed to induce platelet activation and aggregation. Conversely, when the nsPEF duration is decreased, higher electric fields are required for this effect.

For these experiments, freshly isolated human platelets were incubated in a modified Tyrodes buffer containing calcium (as described in, e.g. Dobrydneva and Blackmore (2001)). The equipment used was a Chrono Log model 705 aggregometer. The data was recorded on a chart recorder and also digitized and saved on a computer hard drive. This was achieved by taking the optical signal and amplifying it 100 fold using a Tektronix® 5A22N differential amplifier. The amplified signal was then digitized using a DATAQ DI-194RS serial port data acquisition module and then sent to a P90 pentium computer running on Windows 95. The data were recorded using WinDaq/Lite waveform recording software (DATAQ instruments, Akron OH). The data were then analyzed using WinDaq waveform browser software.

Example 2: Investigation of Activated Platelet Gels Produced by nsPEF for Wound Treatment: 21 New Zealand White rabbits were provided for study. Wounds were created on the backs of the rabbits and treated with platelet gel, platelet poor plasma or left untreated. The dorsal surface of 7 rabbits was shaved and treated with betadine and cleansed with alcohol. General anesthesia was induced by having the animal breathe isofluane 1.5% and oxygen. With the animal under general inhalation anesthesia, 6 cuts were made in the skin of the surgically prepared area using a sterile #10 surgical blade. The wounds were 2mm long, linear, full-thickness incisions inclusive of the dermis and epidermis. One wound was left untreated and one wound was treated with Platelet Poor Plasma ("PPP"). These wounds served as controls. Two wounds were treated with platelet gels activated using nsPEFs (1 pulse, 300ns @ 30kv/cm) and two separate wounds were treated with platelet gel activated with bovine thrombin.

All treatments, including controls, showed a time-dependent decrease in wound areas over four days after wounding, as expected. The biggest wound healing differences were observed 24 hours after wounding. All treatments, including PPP, showed decreased wound areas compared to non-treated controls, indicating enhanced wound healing in all cases. However, wounds treated with platelet gels activated by nsPEFs enhanced healing as effectively as wounds treated with platelet gels activated by thrombin and better than PPP-treated wounds. Thus, platelet gels activated by nsPEFs were at least as effective as platelets activated by thrombin. In some rabbits, nsPEF activated platelet gels showed greater healing potential than thrombin activated platelet gels and differences among the two groups were statistically significant at the 24 hour time point.

The use of platelet gels as a therapeutic agent to enhance wound healing has had a profound effect on surgical and soft tissue wounds. The results demonstrate the use of nsPEF activated gels to replace thrombin activated gels, which carry the potential for untoward effects related to pathophysiological and physiological events.

Platelet gel is thought to enhance wound healing because it creates a more bioactive wound site. An activated platelet aggregation, applied to the wound, increases the level of growth factor signaling proteins and adhesion molecules within the wound site. One beneficial result is the increase in recruitment of cells, including stem cells, to the scaffold formed by the coagulum, and the increase of cell division within the scaffold.

The results suggest that activated platelet gel prepared using nsPEFs is as effective in enhancing healing as platelet gel activated using bovine thrombin. Surprisingly, the area of the surgical wounds treated with platelet gel activated with nsPEFs decreased faster than wounds treated with thrombin activated platelet gels.

Example 3: Effects of Platelet Gel Prepared Using nsPEFs on Heart Wounds: Rabbit hearts were analyzed using Lagendorff preparations in which ischemia was induced in the hearts by cutting off flow through an aortic cannula. Platelet gel or saline was injected into the left ventricular muscle as a means for acute treatment of myocardial infarction.

Fourteen rabbits were euthanized by administering an overdose of xylazine and ketamine, IM. A midline thoracic incision was made and the heart removed. The heart was then placed into a modified Tyrode's solution chilled to 0-4°C and mounted as previously described. *See* Hargrave B and Lattanzio F, Cocaine activates the rennin-angiotensin system in pregnant rabbits and alters the response to ischemia, Cardiovasc Toxicol 2002; 2:91-7. After mounting, a balloon catheter attached to a pressure transducer was inserted into the left ventricle and inflated. Left ventricular systolic and diastolic pressures were recorded every 10 seconds through a polyvinyl catheter using a COBE CDXI11 transducer and Micro-Med 100 Blood Pressure Analyzer (Louisville, KY). The preparation was allowed to beat spontaneously and permitted to equilibrate for 15 minutes prior to initiation of the experimental protocol. PG (0.5 ml) treated with nsPEFs one pulse for 300 ns at 30kv/cm or bovine thrombin or an equal volume of 0.9 % sodium chloride solution,was injected into the muscle layer (myocardium) of the left ventricle. The heart was given 10 minutes to re-stabilize. After the 10 minute re-stabilization period, closing off flow through the aortic cannula was performed to create global ischemia. The ischemia was maintained for 30 minutes with the heart maintained at 37°C. At the end of the 30-minute ischemic period the aortic cannula was re-opened and the heart reperfused for 60 minutes.

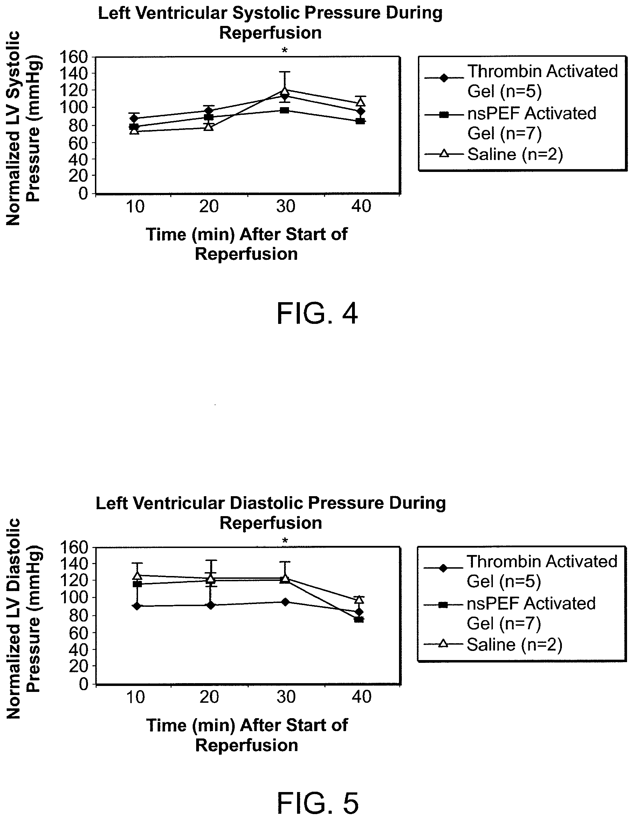

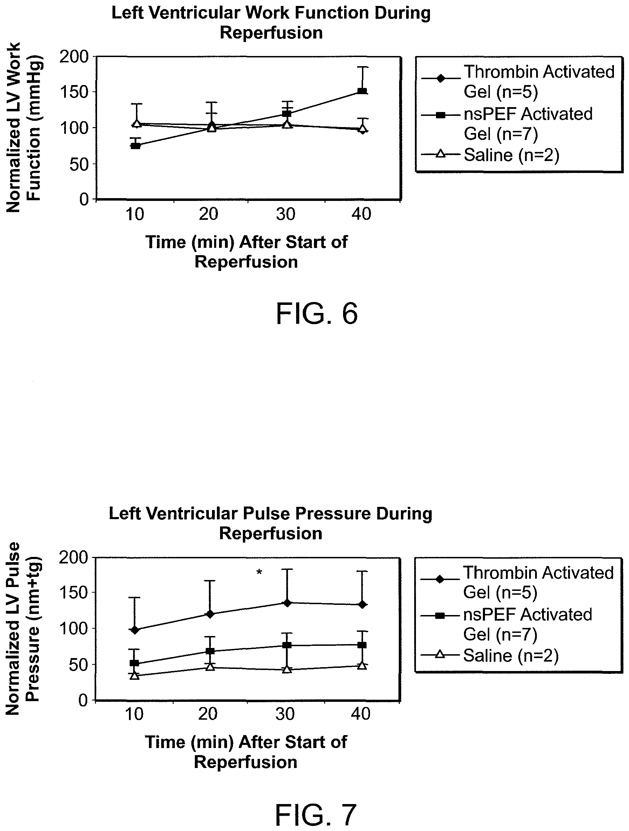

Acute effects of PG activated with nsPEF and thrombin were investigated on left ventricular systolic and diastolic pressure as well as left ventricular work function and pulse pressure. Left ventricular systolic (a = 0.1, p < 0.01) and diastolic (a = 0.05, p<0.03) pressures were higher in the saline treated hearts (control hearts) than in the hearts treated with platelet gel activated with thrombin or nsPEF 30 minutes into reperfusion (Figures 4 and 5). Left ventricular mean pressure was not statistically different in any of the treatments at any of the time points during reperfusion. Forty minutes into reperfusion, left ventricular work function (α = 0.05, p<0.03) was significantly higher in the hearts treated with the nsPEF gel than in the saline or thrombin treated hearts (Figure 6). Heart rate was not significantly different in any of the treatments. Pulse pressure, however, was significantly lower in the saline treated hearts than in the hearts treated with platelet gel activated with thrombin or nsPEF (Figure 7).

Activated platelet gel could, under acute conditions, be used to manipulate the response of the left ventricle of the rabbit heart to ischemic damage as a means of supporting left ventricular mechanical function during ischemia and reperfusion. Platelet gel was injected directly into the myocardium of the rabbit heart prior to exposing the heart to global ischemia and reperfusion. The results observed suggest that even under acute conditions, treatment with platelet gel altered the systolic and diastolic pressure response of the left ventricle to ischemia-reperfusion. Hearts treated with platelet gel had a lower systolic and diastolic pressure than hearts treated with saline (control) 30 minutes into reperfusion.

In clinical situations such as myocardial infarction, changes in cardiac function can be associated with heart failure resulting in a decrease in cardiac output. The decrease in cardiac output results from a decline in stroke volume that may be due to systolic dysfunction, diastolic dysfunction or a combination of the two. Systolic dysfunction refers to impaired ventricular contraction. Contractile dysfunction can result from alterations in signal transduction mechanisms responsible for regulating contraction and/or a loss of viable contracting muscle cells as occurs following acute myocardial infarction. Diastolic dysfunction occurs when the ventricle becomes less compliant, which impairs ventricular filling. One harmful consequence of diastolic and/or systolic dysfunction is a rise in end-diastolic pressure (EDP). Despite the fact that this increase in EDP is a compensatory mechanism designed to maintain cardiac output via the Frank-Starling mechanism, this rise in pressure can cause an increase in left atrial and pulmonary venous pressures, and can lead to pulmonary congestion and edema. Additionally, over months and years these compensatory changes can worsen cardiac function. Activated platelet gel (nsPEF activated or thrombin activated) injected into the left ventricular myocardium blunted the increase in systolic and diastolic pressure, since 30 minutes after the ischemic event and after 30 minutes of reperfusion the diastolic and systolic pressures were higher in the saline (control) treated hearts than in the hearts treated with platelet gel. The lower systolic and diastolic pressures in the platelet gel treated hearts following ischemia may have enhanced ventricular filling and improved or maintained cardiac output. This concept was supported by the fact that pulse pressure, which was used as an indirect measurement of stroke volume, was lower in the saline treated hearts than in the hearts treated with either nsPEF or thrombin activated platelet gel. The elevated systolic and diastolic pressures in the saline treated hearts may suggest less ventricular filling, which leads to reduced stoke volume and cardiac output.

The mechanism by which platelet gel supports the acute pumping function of the left ventricle following ischemia and during reperfusion is unclear. While not wishing to be bound by any particular theory of action, it is possible that activated platelet gel modulates the production of reactive oxygen species (ROS) in the ischemic heart during reperfusion. ROS are highly reactive chemical entities that can exert harmful effects on heart tissue when produced in concentrations that overwhelm the body's inherent antioxidant system. ROS have been shown to have direct electrophysiological effects that contribute to arrhythmias and are implicated in the pathogenesis of post-ischemic myocardial stunning (contractile dysfunction that is reversible). Myocardial cell death after ischemia-reperfusion results from necrosis and apoptosis, which can be activated by ROS. *See* Kevin LG, Novalija E, Stowe DF, Reactive oxygen species as mediators of cardiac injury and protections: The relevance to anesthesia practice, Anesth Analg 2005 101:1275-87. ROS are generated during ischemia and reperfusion. The fact that the hearts were pretreated with platelet gel prior to ischemia-reperfusion may suggest a reduction in the myocyte response to the harmful effects of ROS.

Platelet gel may also lead to expression or increased expression of genes critical to providing energy for the heart. Using an Oligo DEArray® DNA Microarray for Growth Factors, analysis was performed on left ventricular heart tissue after 30 minutes of ischemia and 40 minutes of reperfusion. This microarray profiled the expression of 113 common growth factors (angiogenic GFs, regulators of apoptosis, cell differentiation, embryonic development, and development of specific tissues). Platelet poor plasma ("PPP") was used as a control. Activation of only 2 genes - the bone morphogenic protein10 (BMP-10) and the cytidine deaminase genes - was observed. Under these acute conditions, cytidine deaminase was upregulated. This gene encodes an enzyme involved in pyrimidine salvaging. It is one of several deaminases responsible for maintaining the cellular pyrimidine pool. The early activation of this gene may provide an energy source for the ischemic heart during reperfusion. When ELISA of the left ventricular tissue was performed for the presence of increased PDGF-BB, a growth factor released from platelets, preliminary data suggest the greatest increase in left ventricular tissue treated with platelet gel activated with nsPEFs.

Example 4: Antibacterial Protocol - Bacterial Kill Assay: To compare the ability of activated platelet gel prepared using nsPEFs with activated platelet gel prepared with bovine thrombin to inhibit growth of the clinically relevant bacterium, Staphyiococcus Aureus (ATCC 25923) was placed in a sterile tube containing 4 ml of tryptic soy broth (Sigma-Aldrich, St. Louis, MO) and grown overnight at 37 °C. This protocol generally provided a bacterial concentration of 10⁸ CFU/ml. On day 2 of the experiment, a bacterial sample was serially diluted to a concentration of 10 CFU/ml, treated with 50 µI PPP, phosphate buffered saline (PBS) or platelet gel activated with nsPEF (1 pulse, 30 Kv/cm, 300 ns) or bovine thrombin prepared as previously described. The treated cultures were incubated at 37° C overnight. On day three, 25 ml of the bacterial cultures was placed on a tryptic soy agar plate and incubated for 24 hr at 37° C. The next day the number of colonies formed was counted.

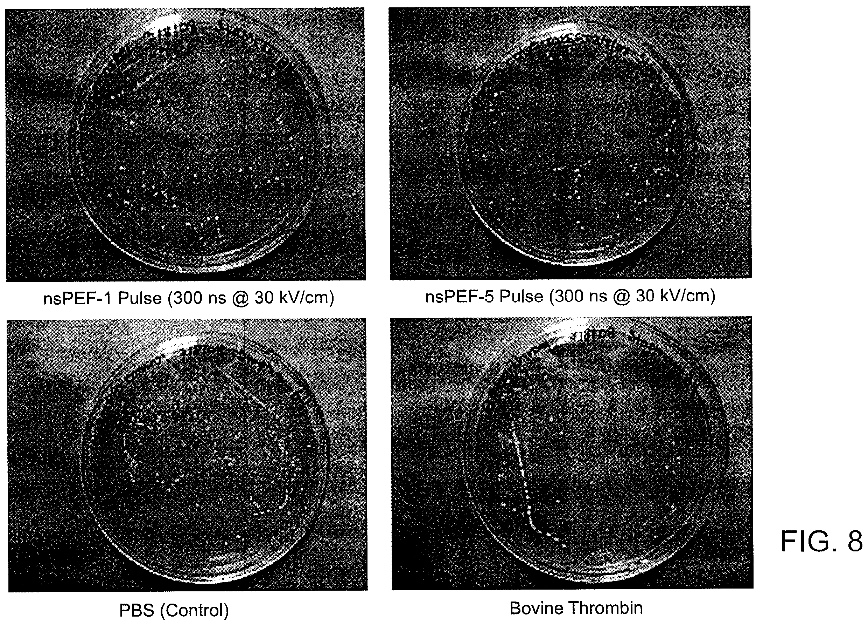

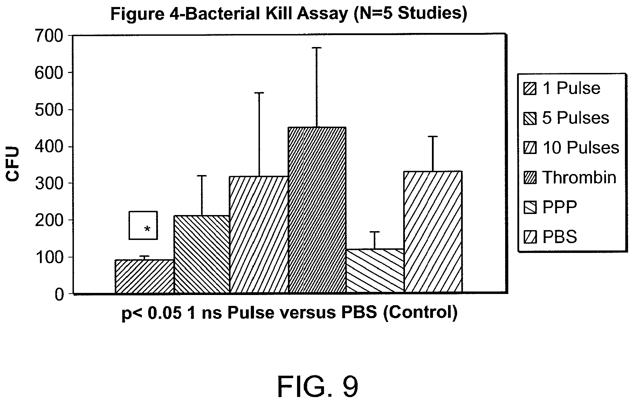

Platelet gels have been reported to exhibit some antibacterial activity. To determine antibacterial activity with platelet gels of the disclosure, effects on Staphylococcus Aureus were investigated (Figures 8 and 9). The platelet gel prepared using one pulse for 300 ns at 30Kv/cm significantly inhibited the growth of Staphylococcus Aureus. Thrombin activated platelet gels had no activity and tended to promoted bacterial growth. In contrast, platelets activated with a single nsPEF treatment exhibited statistically significant antibacterial activity toward the Staphylococcus. Unexpectedly, less antibacterial activity was observed as the pulse number increased. While the change was not statistically significant, there was a tendency for PPP to inhibit bacterial growth.

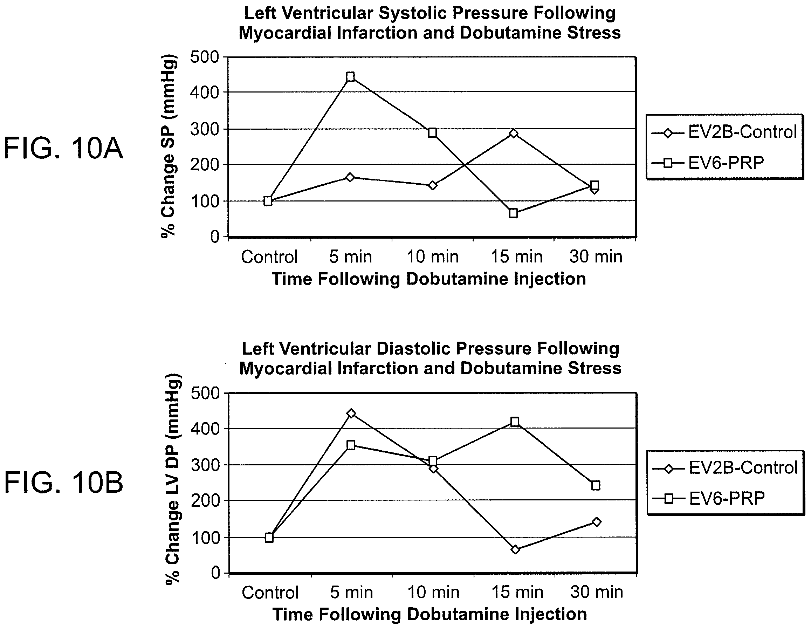

Example 5: In Vivo Experiment: nsPEF-activated platelet gels alters systolic and diastolic pressures and the positive and negative change in pressure over time (dP/dt): In this study a 40% infarct of the left ventricle (through a left thoracotomy) was created by occluding the marginal branch of the left circumflex coronary artery for 10 min. The infarct size was determined by staining the heart with triphenyltetrazol (red) and Blue Heubach-I dispersion dyes. Reperfusion was accomplished by releasing the occlusion. Ten minutes into reperfusion nsPEF-activated platelet gel (0.2 ml) was injected directly into the myocardium of animal EV6. Saline (0.9% [0.2 ml]) was injected into the myocardium of animal EV2B and this animal served as the control. After recovery from anesthesia the animals were returned to their housing units for 14 days. On day 14, each animal was anesthetized and given dobutamine (a positive inotrophic agent) 40 ug/kg intravenously to cause an *in vivo* stress to the heart and left ventricular mechanical function was assessed. Dobutamine was given intravenously over a 3 min period, starting with 5ug/kg and increasing the dose every minute to 10, 20 and finally 40ug/kg. Data in Figures 10A-D were normalized to a control of 100%.

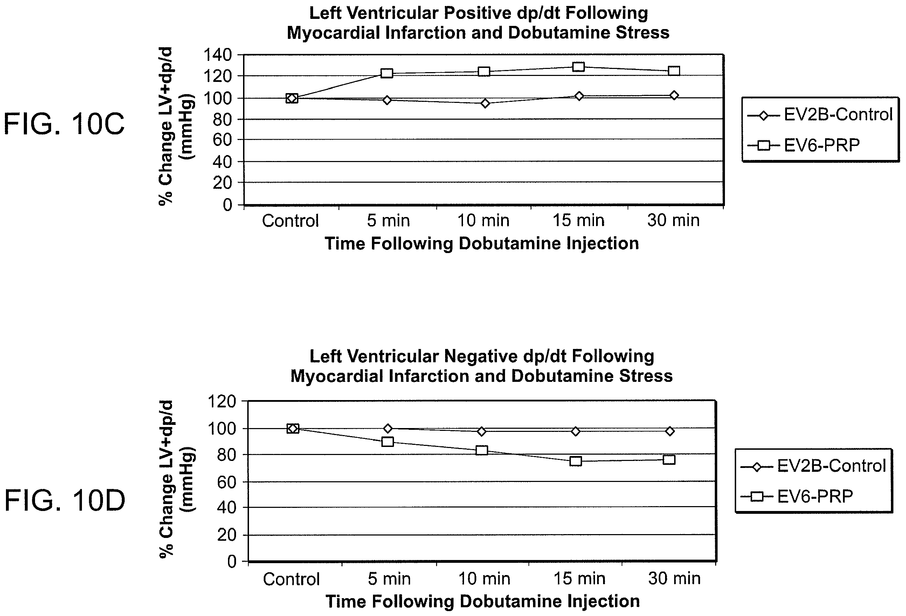

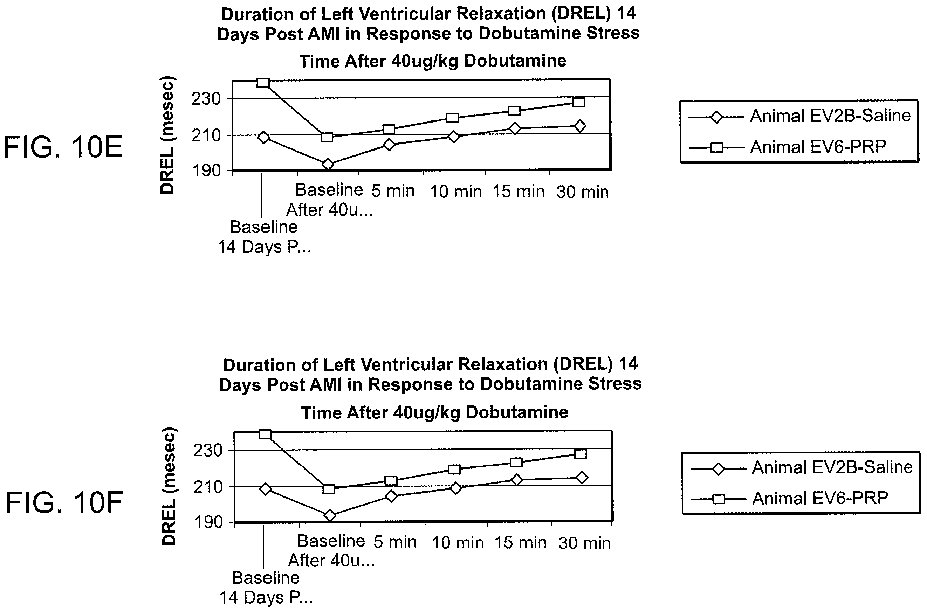

Positive and Negative dP/dt: The nsPEF-activated platelet gel treated heart responded to the dobutamine induced stress with the tendency to increase left ventricular positive dP/dt (a measure of the ability of the left ventricle to pump effectively (Fig 10C) 14 days after AMI while the tendency of the saline treated heart was to maintain dP/dt fairly constant. Just as important, in the nsPEF-activated platelet gel treated heart there was the tendency for negative dP/dt (a measure of how well the heart left ventricle relaxes) to decrease in response to the dobutamine stress whereas the saline treated heart was unable to respond (Fig 10D). This data is consistent with *in vitro* results and again suggest a possible role for nsPEF-activated platelet gel in mitigating left ventricular pressure following AMI. Changes in heart rate (HR), the duration of left ventricular contraction (DCON) and relaxation (DREL), were also analyzed. There was a tendency for a lower HR (HR was 10% lower) in the PRP (EV6) treated animal than in the animal treated with saline (EV2B). Physiologically, this suggests that the duration of the cardiac cycle in the saline heart is shorter than in the heart treated with nsPEF-activated platelet gel Generally, when the cardiac cycle is shortened, time spent in diastole (filling) is reduced. Of interest is that the HR in the nsPEF-activated platelet gel treated animal, when stressed with dobutamine showed a tendency not to increase as much and to decrease faster than HR in the saline treated animal. Additionally, 30 min after the stressor the tendency was for a lower HR (7% lower) in the nsPEF-activated platelet gel treated animal than the saline treated animal suggesting a longer diastole for the nsPEF-activated platelet gel treated heart. Analysis of the duration of contraction (DCON) and relaxation of the left ventricle in both animals was performed. Fourteen days post AMI DCON was comparable in both animals. However, under stress the hearts behaved differently. Thirty min after the stressor there was a tendency for a greater DCON (20% greater, Fig. 10E) and DREL (6% longer, Fig. 10F) in the nsPEF-activated platelet gel treated than in the saline treated animal suggesting the a longer duration of the cardiac cycle, more time diastole and better cardiac filling in. Consistent with these data are the data in Fig. 10E which shows that in the nsPEF-activated platelet gel treated animal the tendency is toward a longer duration of LV relaxation than in the saline treated animal.

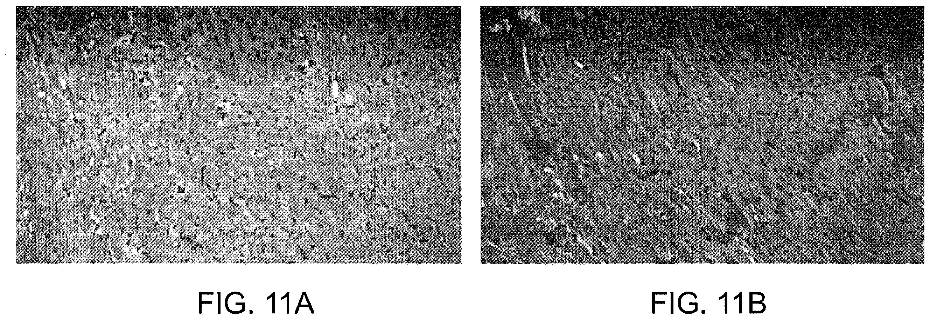

Example 6: Microscopic Proof of Concept-Microscopic inspection of the heart from a rabbit treated with saline (Fig. 11A, n=1 heart) or nsPEF-activated platelet gel (Fig 11B, n=1): A 40% infarct, *in vivo,* of the left ventricle (through a left thoracotomy) was created by occluding the marginal branch of the left circumflex coronary artery for 10 min. Reperfusion was accomplished by releasing the occlusion. nsPEF platelet gel was activated with one nsPEF having a pulse length of 300 ns and an electrical field strength of 30kV/cm. Ten minutes into reperfusion, the nsPEF-activated platelet gel (0.2 ml) or 0.9% saline (0.2 ml) was injected into the myocardium. The chest was closed and the animal returned to its housing facility for 14 days. On day 14 the animal was given dobutamine as previously described to cause an *in vivo* stress to the infarcted heart so that the mechanical function could be assessed. The heart was then removed and stained with Hematoxylin and Eosin stain. The heart treated with saline (Fig 11A) had a moth eaten appearance, necrosis, and extensive vacuolization (formation of vacuoles) of myofibrils, a pattern reminiscent of hypertrophic cardiomyopathy. The heart treated with nsPEF-activated platelet gel (Fig. 11B) had only mild necrosis, minimal vacuolization and minimum myocyte disarray.

Example 7: Microarray analysis of left ventricular heart tissue treated with nsPEF-activated platelet gel or platelet poor plasma (PPP): The CDA gene encodes an enzyme involved in pyrimidine salvaging. It is one of several deaminases responsible for maintaining the cellular pyrimidine pool which serves as an energy source for the heart. The genes for both IL-6 and IL-11 were also activated in the nsPEF-activated platelet gel treated hearts. Although the IL-6 family of cytokines has proinflammatory properties evidence suggest it also activates signal transducer and activator of transcription (STAT) proteins. STAT-3 is thought to contribute to cardio-protection and vessel formation since ablation ofd the STAT-3 gene leads to cardiac heart failure and impaired capillary growth. The IL-11 gene was also activated. It functions as an immunoregulator and has anti-inflammatory effects via its ability to regulate effector cell function and prevents reperfusion injury in intestine. IL-11 is increased in hearts treated with nsPEF-activated platelet gel (Table 1) and therefore may serve to prevent ischemia reperfusion injury in the heart as well.

**Table 1. Oligo DEArray® DNA Microarray**

| **Gene** | **Right Ventricle PRP/PPP ratio** | **Left Ventricle PRP/PPP Ratio** |
|---|---|---|
| Bone morphogenetic protein 10 (Bmp 10) | 2.0 | 7.0 |
| Cytidine deaminase (Cda) | | 2.65 |
| Growth Differentiation factor 11 (Gdf 11) | 3.46 | |
| Kit Ligand (Kitl) | 5.14 | |
| Interleukin 11 (IL 11) | 2.38 | |
| Interleukin 6 (IL 6) | 3.46 | |
| Tyrosine kinase, non-receptor 1 (Tnk1) | 2.12 | |

Although IL-11 has been reported to be expressed in cardiac myocytes (Ancly 2002) the treatment with nsPEF-activated platelet gel which contains IL-11 released from the concentrated/activated platelets may help to explain our preliminary results which suggest that in both the Langendorff heart and the in vivo AMI left ventricular mechanical function is better in nsPEF-activated platelet gel treated hearts. Therefore, nsPEF-activated platelet gel could be a therapeutic strategy for AMI. IL-11, like IL-6 activates STAT-3 and ERK ½ in cardiac myocytes, causes cell elongation and confers a resistance to cell death induced by hydrogen peroxide. C-Kit-ligand gene, also known as stem cell factor has been shown to be activated by Nkx2-5 transcription factor and regulates the cardiac progenitor cell population. Tnk1 is a receptor tyrosine kinase which has a high affinity cell surface receptors for many polypeptide growth factors, cytokines and hormones. All of these genes were activated in the nsPEF-activated platelet gel treated heart but not in the control tissue and may provide a clue as to why there is the tendency for the mechanical performance of the nsPEF-activated platelet gel] treated hearts to be better than that of the saline treated hearts.

There are several advantages to using nsPEFs as a platelet activator in the preparation of aggregates or platelet gels. Use of nsPEFs provides an effective and safe means to release "healing" factors (proteins) at the site of a wound; it provides the use of a non-chemical agonist that does not have the potential to induce untoward systemic effects. Growth factors that are reported to be present in platelet gel include, but are not limited to, interlukin 1 beta, transforming growth factor beta, transforming growth factor alpha, FGF, EGF, platelet derived growth factor, and insulin-like growth factor, all of which support the concept that platelet concentrates can mediate healing.

Untoward effects can be associated with thrombin and most chemical agonists. For example, bovine thrombin has been associated with severe post-operative bleeding stemming from the development of cross-reactive anti-bovine antibodies that inhibit human coagulation factors V. Furthermore, exposure to contaminated bovine derived prions, which are highly robust and infectious proteins, has been associated with the etiology of variant Creutzfeld-Jacob disease in humans and diseases of the central nervous system. Thrombin also promotes tumor cell seeding and adhesion to the endothelium and extracellular matrix, thus enhancing the metastatic capacity of tumors. Human thrombin carries the potential risk for transmission of viral particles. Thus, nsPEFs make available a means to heal wounds without the potential to transmit infectious agents or cause untoward inflammation response.

The foregoing detailed description includes many specific details. The inclusion of such detail is for the purpose of illustration only and should not be understood to limit the invention. In addition, features in one embodiment may be combined with features in other embodiments of the invention. Various changes may be made without departing from the scope of the invention as defined in the following claims. In addition, all non-priority patents and other references cited herein are indicative of the level of skill in the art.

## Claims

1. A platelet gel for use in a method of treating systolic dysfunction and/or diasystolic dysfunction of a heart in a subject, wherein the platelet gel is obtained by a method comprising:
concentrating platelets; and
activating the concentrated platelets by applying at least one electrical pulse to the concentrated platelets,
wherein the electrical pulse has a duration of at least about 100 picoseconds and less than about 1 microsecond and an electric field strength of at least about 10 kV/cm and less than about 350 kV/cm.

2. A bandage or a tissue repair matrix for use in a method of treating systolic dysfunction and/or diasystolic dysfunction of a heart in a subject, the bandage or tissue repair matrix comprising a platelet gel, the platelet gel being obtained by a method comprising:
concentrating platelets; and
activating the concentrated platelets by applying at least one electrical pulse to the concentrated platelets,
wherein the electrical pulse has a duration of at least about 100 picoseconds and less than about 1 microsecond and an electric field strength of at least about 10 kV/cm and less than about 350 kV/cm.

3. The bandage for use or tissue repair matrix for use of claim 1 or claim 2, wherein the platelets are autologous.

4. The platelet gel for use, bandage for use or tissue repair matrix for use of any preceding claim, wherein the electrical pulse has a duration of 300 ns and the electrical field strength is 30 kV/cm.

5. The platelet gel for use, bandage for use, or tissue repair matrix for use of any preceding claim, wherein the method of treating systolic dysfunction and/or diastolic dysfunction comprises modulating production of reactive oxygen species (ROS).

6. The platelet gel for use, bandage for use, or tissue repair matrix for use of any preceding claim, wherein the method of treating systolic dysfunction and/or diastolic dysfunction comprises a method of treating acute changes in systolic and diastolic pressures in the heart after an ischemic event, optionally wherein the ischemic event is ischemia-reperfusion.

7. The platelet gel for use, bandage for use, or tissue repair matrix for use of claim 6, wherein the method of treating acute changes in systolic and diastolic pressures in the heart involves treating the left ventricle of the heart.

8. The platelet gel for use, bandage for use, or tissue repair matrix for use of any preceding claim, wherein systolic dysfunction and/or diastolic dysfunction is due to myocardial infarction.

9. The platelet gel for use, bandage for use, or tissue repair matrix for use of any preceding claim, wherein the method of treating systolic dysfunction and/or diastolic dysfunction comprises a step of injecting the activated platelets directly into the myocardium, optionally comprises a step of injecting the activated platelets directly into the left ventricular myocardium.

10. The platelet gel for use, bandage for use, or tissue repair matrix for use of any preceding claim, wherein the method of treating systolic dysfunction and/or diastolic dysfunction comprises applying at least two electrical pulses, optionally applying at least five electrical pulses, optionally applying at least ten electrical pulses.

11. The platelet gel for use, bandage for use, or tissue repair matrix for use of any preceding claim, wherein the method of obtaining the platelet gel further comprises applying at least two electrical pulses and no more than 10 electrical pulses.

12. The platelet gel for use, bandage for use, or tissue repair matrix for use of any preceding claim, wherein the at least one electric pulse is administered by a pulse generator configured to produce at least one electrical pulse having a duration of at least about 100 picoseconds to about 1 microsecond and an electric field strength of at least about 10 kV/cm to about 350 kV/cm, optionally in which the pulse generator is configured to produce at least one electrical pulse having a duration of at least about 100 picoseconds to about 1 microsecond and an electric field strength of at least about 10 kV/cm to about 125 kV/cm.

13. The platelet gel for use, bandage for use, or tissue repair matrix for use of any preceding claim, wherein the at least one electric pulse is administered by a pulse generator configured to produce at least one electrical pulse having a duration of at least about 10 nanoseconds to about 100 nanoseconds and an electric field strength of at least about 10 kV/cm to about 30 kV/cm, optionally in which the pulse generator is configured to produce at least one electrical pulse having a duration of at least about 10 nanoseconds to about 100 nanoseconds and an electric field strength of at least about 10 kV/cm to about 125 kV/cm, and optionally in which the pulse generator is configured to produce at least one electrical pulse having a duration of about 60 nanoseconds and an electric field strength of about 30 kV/cm.

14. The platelet gel for use, bandage for use, or tissue repair matrix for use of any preceding claim, wherein the at least one electric pulse is administered by a pulse generator configured to produce at least one electrical pulse having a duration of at least about 100 picoseconds to about 1 microsecond and an electric field strength of at least about 10 kV/cm to about 125 kV/cm, optionally in which the pulse generator is configured to produce at least one electrical pulse having a duration of about 300 nanoseconds and an electric field strength of about 30 kV/cm.

15. The platelet gel for use, bandage for use, or tissue repair matrix for use of any preceding claim, wherein the platelets are pulsed outside a human or an animal body.

## Patentansprüche

1. Thrombozyten-Gel zur Verwendung bei einem Verfahren zur Behandlung von systolischer Dysfunktion und/oder diastolischer Dysfunktion des Herzes in einem Subjekt, wobei das Thrombozyten-Gel durch ein Verfahren erhalten ist, das umfasst:
Konzentrieren von Thrombozyten; und
Aktivieren der konzentrierten Thrombozyten durch Anlegen wenigstens eines elektrischen Pulses an die konzentrierten Thrombozyten,
wobei der elektrische Puls eine Dauer von wenigstens etwa 100 Pikosekunden und weniger als etwa 1 Mikrosekunde und eine elektrische Feldstärke von wenigstens etwa 10 kV/cm und weniger als etwa 350 kV/cm aufweist.

2. Verband oder Gewebereparaturmatrix zur Verwendung bei einem Verfahren zur Behandlung von systolischer Dysfunktion und/oder diastolischer Dysfunktion des Herzes in einem Subjekt, wobei der Verband oder die Gewebereparaturmatrix ein Thrombozyten-Gel umfasst, wobei das Thrombozyten-Gel durch ein Verfahren erhalten ist, das umfasst:
Konzentrieren von Thrombozyten; und
Aktivieren der konzentrierten Thrombozyten durch Anlegen wenigstens eines elektrischen Pulses an die konzentrierten Thrombozyten,
wobei der elektrische Puls eine Dauer von wenigstens etwa 100 Pikosekunden und weniger als etwa 1 Mikrosekunde und eine elektrische Feldstärke von wenigstens etwa 10 kV/cm und weniger als etwa 350 kV/cm aufweist.

3. Verband oder Gewebereparaturmatrix zur Verwendung gemäß Anspruch 1 oder Anspruch 2, wobei die Thrombozyten autolog sind.

4. Thrombozyten-Gel zur Verwendung, Verband zur Verwendung oder Gewebereparaturmatrix zur Verwendung gemäß einem der vorstehenden Ansprüche, wobei der elektrische Puls eine Dauer von 300 ns aufweist und die elektrische Feldstärke 30 kV/cm beträgt.

5. Thrombozyten-Gel zur Verwendung, Verband zur Verwendung oder Gewebereparaturmatrix zur Verwendung gemäß einem der vorstehenden Ansprüche, wobei das Verfahren zur Behandlung von systolischer Dysfunktion und/oder diastolischer Dysfunktion Modulieren der Produktion von reaktiven Sauerstoffspezies (ROS) umfasst.

6. Thrombozyten-Gel zur Verwendung, Verband zur Verwendung oder Gewebereparaturmatrix zur Verwendung gemäß einem der vorstehenden Ansprüche, wobei das Verfahren zur Behandlung von systolischer Dysfunktion und/oder diastolischer Dysfunktion ein Verfahren zur Behandlung von akuten Veränderungen des systolischen und des diastolischen Drucks in dem Herz nach einem ischämischen Ereignis umfasst, wobei das ischämische Ereignis gegebenenfalls Ischämie-Reperfusion ist.

7. Thrombozyten-Gel zur Verwendung, Verband zur Verwendung oder Gewebereparaturmatrix zur Verwendung gemäß Anspruch 6, wobei das Verfahren zur Behandlung von akuten Veränderungen des systolischen und des diastolischen Drucks in dem Herz mit der Behandlung des linken Ventrikels des Herzes verbunden ist.

8. Thrombozyten-Gel zur Verwendung, Verband zur Verwendung oder Gewebereparaturmatrix zur Verwendung gemäß einem der vorstehenden Ansprüche, wobei die systolische Dysfunktion und/oder diastolische Dysfunktion von Myokardinfarkt verursacht ist.

9. Thrombozyten-Gel zur Verwendung, Verband zur Verwendung oder Gewebereparaturmatrix zur Verwendung gemäß einem der vorstehenden Ansprüche, wobei das Verfahren zur Behandlung von systolischer Dysfunktion und/oder diastolischer Dysfunktion einen Schritt des Einspritzens der aktivierten Thrombozyten direkt in das Myokard umfasst, gegebenenfalls einen Schritt des Einspritzens der aktivierten Thrombozyten direkt in das linksventrikuläre Myokard umfasst.

10. Thrombozyten-Gel zur Verwendung, Verband zur Verwendung oder Gewebereparaturmatrix zur Verwendung gemäß einem der vorstehenden Ansprüche, wobei das Verfahren zur Behandlung von systolischer Dysfunktion und/oder diastolischer Dysfunktion Anlegen von wenigstens zwei elektrischen Pulsen, gegebenenfalls Anlegen von wenigstens fünf elektrischen Pulsen, gegebenenfalls Anlegen von wenigstens zehn elektrischen Pulsen, umfasst.

11. Thrombozyten-Gel zur Verwendung, Verband zur Verwendung oder Gewebereparaturmatrix zur Verwendung gemäß einem der vorstehenden Ansprüche, wobei das Verfahren zum Erhalten des Thrombozyten-Gels ferner Anlegen von wenigstens zwei elektrischen Pulsen und nicht mehr als 10 elektrischen Pulsen umfasst.

12. Thrombozyten-Gel zur Verwendung, Verband zur Verwendung oder Gewebereparaturmatrix zur Verwendung gemäß einem der vorstehenden Ansprüche, wobei der wenigstens eine elektrische Puls von einem Pulsgenerator verabreicht wird, der dafür gestaltet ist, wenigstens einen elektrischen Puls mit einer Dauer von wenigstens etwa 100 Pikosekunden bis etwa 1 Mikrosekunde und einer elektrischen Feldstärke von wenigstens etwa 10 kV/cm bis etwa 350 kV/cm zu erzeugen, wobei der Pulsgenerator gegebenenfalls dafür gestaltet ist, wenigstens einen elektrischen Puls mit einer Dauer von wenigstens etwa 100 Pikosekunden bis etwa 1 Mikrosekunde und einer elektrischen Feldstärke von wenigstens etwa 10 kV/cm bis etwa 125 kV/cm zu erzeugen.

13. Thrombozyten-Gel zur Verwendung, Verband zur Verwendung oder Gewebereparaturmatrix zur Verwendung gemäß einem der vorstehenden Ansprüche, wobei der wenigstens eine elektrische Puls von einem Pulsgenerator verabreicht wird, der dafür gestaltet ist, wenigstens einen elektrischen Puls mit einer Dauer von wenigstens etwa 10 Nanosekunden bis etwa 100 Nanosekunden und einer elektrischen Feldstärke von wenigstens etwa 10 kV/cm bis etwa 30 kV/cm zu erzeugen, wobei der Pulsgenerator gegebenenfalls dafür gestaltet ist, wenigstens einen elektrischen Puls mit einer Dauer von wenigstens etwa 10 Nanosekunden bis etwa 100 Nanosekunden und einer elektrischen Feldstärke von wenigstens etwa 10 kV/cm bis etwa 125 kV/cm zu erzeugen, und wobei der Pulsgenerator gegebenenfalls dafür gestaltet ist, wenigstens einen elektrischen Puls mit einer Dauer von etwa 60 Nanosekunden und einer elektrischen Feldstärke von etwa 30 kV/cm zu erzeugen.

14. Thrombozyten-Gel zur Verwendung, Verband zur Verwendung oder Gewebereparaturmatrix zur Verwendung gemäß einem der vorstehenden Ansprüche, wobei der wenigstens eine elektrische Puls von einem Pulsgenerator verabreicht wird, der dafür gestaltet ist, wenigstens einen elektrischen Puls mit einer Dauer von wenigstens etwa 100 Pikosekunden bis etwa 1 Mikrosekunde und einer elektrischen Feldstärke von wenigstens etwa 10 kV/cm bis etwa 125 kV/cm zu erzeugen, wobei der Pulsgenerator gegebenenfalls dafür gestaltet ist, wenigstens einen elektrischen Puls mit einer Dauer von etwa 300 Nanosekunden und einer elektrischen Feldstärke von etwa 30 kV/cm zu erzeugen.

15. Thrombozyten-Gel zur Verwendung, Verband zur Verwendung oder Gewebereparaturmatrix zur Verwendung gemäß einem der vorstehenden Ansprüche, wobei die Thrombozyten außerhalb eines menschlichen Körpers oder Tierkörpers pulsbehandelt werden.

## Revendications

1. Gel de plaquettes pour utilisation dans un procédé de traitement d'un trouble systolique et/ou d'un trouble diastolique d'un cœur chez un sujet, le gel de plaquettes étant obtenu par un procédé comprenant :
la concentration de plaquettes ; et
l'activation des plaquettes concentrées par application d'au moins une impulsion électrique aux plaquettes concentrées,
l'impulsion électrique ayant une durée d'au moins environ 100 picosecondes et inférieure à environ 1 microseconde et une intensité de champ électrique d'au moins environ 10 kV/cm et inférieure à environ 350 kV/cm.

2. Pansement ou matrice de réparation de tissu pour utilisation dans un procédé de traitement d'un trouble systolique et/ou d'un trouble diastolique d'un cœur chez un sujet, le pansement ou la matrice de réparation de tissu comprenant un gel de plaquettes, le gel de plaquettes étant obtenu par un procédé comprenant :
la concentration de plaquettes ; et
l'activation des plaquettes concentrées par application d'au moins une impulsion électrique aux plaquettes concentrées,
l'impulsion électrique ayant une durée d'au moins environ 100 picosecondes et inférieure à environ 1 microseconde et une intensité de champ électrique d'au moins environ 10 kV/cm et inférieure à environ 350 kV/cm.

3. Pansement pour utilisation ou matrice de réparation de tissu pour utilisation selon la revendication 1 ou la revendication 2, les plaquettes étant autologues.

4. Gel de plaquettes pour utilisation, pansement pour utilisation ou matrice de réparation de tissu pour utilisation selon l'une quelconque des revendications précédentes, l'impulsion électrique ayant une durée de 300 ns et l'intensité de champ électrique étant de 30 kV/cm.

5. Gel de plaquettes pour utilisation, pansement pour utilisation, ou matrice de réparation de tissu pour utilisation selon l'une quelconque des revendications précédentes, le procédé de traitement d'un trouble systolique et/ou d'un trouble diastolique comprenant la modulation de la production d'espèces réactives de l'oxygène (ROS).

6. Gel de plaquettes pour utilisation, pansement pour utilisation, ou matrice de réparation de tissu pour utilisation selon l'une quelconque des revendications précédentes, le procédé de traitement d'un trouble systolique et/ou d'un trouble diastolique comprenant un procédé de traitement de changements aigus des pressions systolique et diastolique dans le cœur après un événement ischémique, l'événement ischémique étant facultativement une ischémie-reperfusion.

7. Gel de plaquettes pour utilisation, pansement pour utilisation, ou matrice de réparation de tissu pour utilisation selon la revendication 6, le procédé de traitement de changements aigus des pressions systolique et diastolique dans le cœur mettant en œuvre le traitement du ventricule gauche du cœur.

8. Gel de plaquettes pour utilisation, pansement pour utilisation, ou matrice de réparation de tissu pour utilisation selon l'une quelconque des revendications précédentes, le trouble systolique et/ou le trouble diastolique étant dû à un infarctus du myocarde.

9. Gel de plaquettes pour utilisation, pansement pour utilisation, ou matrice de réparation de tissu pour utilisation selon l'une quelconque des revendications précédentes, le procédé de traitement d'un trouble systolique et/ou d'un trouble diastolique comprenant une étape d'injection des plaquettes activées directement dans le myocarde, comprenant facultativement une étape d'injection des plaquettes activées directement dans le myocarde ventriculaire gauche.

10. Gel de plaquettes pour utilisation, pansement pour utilisation, ou matrice de réparation de tissu pour utilisation selon l'une quelconque des revendications précédentes, le procédé de traitement d'un trouble systolique et/ou d'un trouble diastolique comprenant l'application d'au moins deux impulsions électriques, facultativement l'application d'au moins cinq impulsions électriques, facultativement l'application d'au moins dix impulsions électriques.

11. Gel de plaquettes pour utilisation, pansement pour utilisation, ou matrice de réparation de tissu pour utilisation selon l'une quelconque des revendications précédentes, le procédé d'obtention du gel de plaquettes comprenant en outre l'application d'au moins deux impulsions électriques et pas plus de 10 impulsions électriques.

12. Gel de plaquettes pour utilisation, pansement pour utilisation, ou matrice de réparation de tissu pour utilisation selon l'une quelconque des revendications précédentes, dans lesquels l'au moins une impulsion électrique est administrée par un générateur d'impulsions configuré pour produire au moins une impulsion électrique ayant une durée d'au moins environ 100 picosecondes à environ 1 microseconde et une intensité de champ électrique d'au moins environ 10 kV/cm à environ 350 kV/cm, facultativement dans lesquels le générateur d'impulsions est configuré pour produire au moins une impulsion électrique ayant une durée d'au moins environ 100 picosecondes à environ 1 microseconde et une intensité de champ électrique d'au moins environ 10 kV/cm à environ 125 kV/cm.

13. Gel de plaquettes pour utilisation, pansement pour utilisation, ou matrice de réparation de tissu pour utilisation selon l'une quelconque des revendications précédentes, dans lesquels l'au moins une impulsion électrique est administrée par un générateur d'impulsions configuré pour produire au moins une impulsion électrique ayant une durée d'au moins environ 10 nanosecondes à environ 100 nanosecondes et une intensité de champ électrique d'au moins environ 10 kV/cm à environ 30 kV/cm, facultativement dans lesquels le générateur d'impulsions est configuré pour produire au moins une impulsion électrique ayant une durée d'au moins environ 10 nanosecondes à environ 100 nanosecondes et une intensité de champ électrique d'au moins environ 10 kV/cm à environ 125 kV/cm, et facultativement dans lesquels le générateur d'impulsions est configuré pour produire au moins une impulsion électrique ayant une durée d'environ 60 nanosecondes et une intensité de champ électrique d'environ 30 kV/cm.

14. Gel de plaquettes pour utilisation, pansement pour utilisation, ou matrice de réparation de tissu pour utilisation selon l'une quelconque des revendications précédentes, dans lesquels l'au moins une impulsion électrique est administrée par un générateur d'impulsions configuré pour produire au moins une impulsion électrique ayant une durée d'au moins environ 100 picosecondes à environ 1 microseconde et une intensité de champ électrique d'au moins environ 10 kV/cm à environ 125 kV/cm, facultativement dans lesquels le générateur d'impulsions est configuré pour produire au moins une impulsion électrique ayant une durée d'environ 300 nanosecondes et une intensité de champ électrique d'environ 30 kV/cm.

15. Gel de plaquettes pour utilisation, pansement pour utilisation, ou matrice de réparation de tissu pour utilisation selon l'une quelconque des revendications précédentes, les plaquettes étant pulsées à l'extérieur d'un corps humain ou animal.
